# EUROPEAN PATENT SPECIFICATION

(11) **EP 4 403 172 B1**
(45) Date of publication and mention of the grant of the patent: **11.11.2026**
(21) Application number: 24179248.0
(22) Date of filing: 22.12.2010
(51) Int. Cl.: A61K 9/70, A61K 31/381

(54) **POLYVINYLPYRROLIDONE FOR THE STABILIZATION OF A SOLID DISPERSION OF THE NON-CRYSTALLINE FORM OF ROTIGOTINE**
POLYVINYLPYRROLIDONE ZUR STABILISIERUNG EINER FESTEN DISPERSION DER NICHTKRISTALLINEN FORM VON ROTIGOTIN
POLYVINYLPYRROLIDONE POUR LA STABILISATION D'UNE DISPERSION SOLIDE DE LA FORME NON CRISTALLINE DE ROTIGOTINE

(30) Priority: 22.12.2009 US 28930209 P
(43) Date of publication of application: 24.07.2024
(62) Document number(s) of the earlier application(s) in accordance with Art. 76 EPC: 17185284.1 / 3 257 504; 10796399.3 / 2 515 887
(73) Proprietor: UCB Biopharma SRL, 1070 Brussels (BE); LTS LOHMANN Therapie-Systeme AG, 56626 Andernach (DE)
(72) Inventor: Wolff, Hans-Michael, 40789 Monheim (DE); Arth, Christoph, 40789 Monheim (DE); Quere, Luc, 1420 Braine-I'Allued (BE); Müller, Walter, 1420 Braine-I'Allued (BE)
(74) Representative: Hoffmann Eitle

(56) References cited:
- EP-A1- 1 386 605
- EP-A1- 1 669 063
- EP-A1- 2 177 217
- WO-A1-03/092677

## Description

### FIELD

The present invention relates to a transdermal therapeutic system comprising a stabilized solid dispersion as a self adhesive matrix comprising rotigotine free base and polyvinylpyrrolidone in a weight ratio of 9:6, which is characterized by an increased long term storage stability due to the reduced formation of rotigotine crystals.

### BACKGROUND

Rotigotine is the International Non-Proprietary Name (INN) of the compound (-)-5,6,7,8-tetrahydro-6-[propyl-[2-(2-thienyl)ethyl]-amino]-1-naphthalenol having the structure shown below

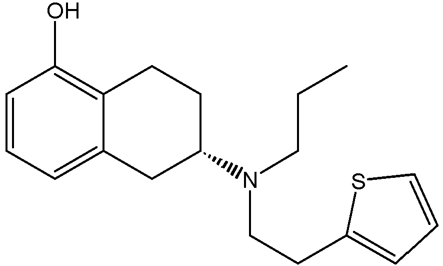

Two crystalline forms of rotigotine are presently known: polymorphic form I and polymorphic form II (WO 2009/068520). They can be differentiated by their respective physicochemical parameters, i.e. differing powder X-ray diffraction spectra, Raman spectra and melting points. At room temperature, the crystalline polymorphic form II is more stable than form I, which in turn is more stable than the amorphous form of rotigotine.

Rotigotine is a non-ergolinic D1/D2/D3 dopamine agonist that resembles dopamine structurally and has a similar receptor profile but a higher receptor affinity.

In contrast to other non-ergolinic dopamine agonists, rotigotine has significant D1 activity, which may contribute to a more physiological action.

In contrast to ergolinic compounds, rotigotine has a very low affinity for 5 HT₂B receptors and thus a low risk of inducing fibrosis.

Actions on non-dopaminergic receptors (such as 5-HT₁A agonism and A_{2B} antagonism) may contribute to other beneficial effects, such as antidyskinetic activity, neuroprotective activity and antidepressive effects.

Rotigotine is disclosed as active agent for treating patients suffering from Parkinson's disease (described in WO 2002/089777), Parkinson's plus syndrome (described in WO 2005/092331), depression (described in WO 2005/009424) and the restless-legs syndrome (described in WO 2003/092677) as well as for the treatment or prevention of dopaminergic neuron loss (described in WO 2005/063237) and treatment of pain (PCT/EP2007/005381).

Known pharmaceutical compositions containing rotigotine comprise a transdermal therapeutic system or patch (TTS) (described *inter alia* in WO 99/49852), a depot form (described in WO 02/15903), an iontophoretic device (described in WO 2004/050083) and an intranasal formulation (described in WO 2005/063236). Transdermal therapeutic systems or patches containing rotigotine and polyvinylpyrrolidone can be prepared as solvent-based systems (described in WO 03/092677, EP 1 386 605 A1 or EP 2 177 217 A1) or as hot melt-based systems (described in EP 1 669 063).

To date, various TTS for the administration of amine functional drugs, such as rotigotine and many others, have been described.

WO 94/07468 discloses a TTS containing rotigotine hydrochloride as active substance in a two-phase matrix, which is essentially formed by a hydrophobic polymer material as the continuous phase and a disperse hydrophilic phase contained therein and mainly containing the drug and hydrated silica. The silica is said to enhance the maximum possible loading of the TTS with the hydrophilic salt. Moreover, the formulation of WO 94/07468 usually contains additional hydrophobic solvents, permeation promoting substances, dispersing agents and, in particular, an emulsifier, which is required to emulsify the aqueous solution of the active component in the lipophilic polymer phase. A TTS prepared by using such a system has been tested in healthy subjects and Parkinson's patients. However, no satisfactory drug plasma levels were achieved.

Various further TTS have been described in WO 99/49852. Various further transdermal therapeutic systems have been described in WO 99/49852. The TTS used in this patent application comprise a backing layer, inert with respect to the constituents of the matrix, a self-adhesive matrix layer containing an effective quantity of rotigotine or rotigotine hydrochloride and a protective film which is to be removed before use. The matrix system is composed of a non-aqueous polymer adhesive system, based on acrylate or silicone.

Further TTS for the delivery of rotigotine are for example disclosed in EP 1 256 339 and in WO 2004/012730. These systems utilize a mixture of at least one high tack and at least one medium tack amine-compatible silicone pressure sensitive adhesive as the main adhesive components of the self adhesive matrix. They provide for better flux rates and sufficient rotigotine plasma levels.

Despite the existence of TTS comprising rotigotine in the prior art, there remains room for improvement in such TTS, and in processes for making them.

Crucial pharmaceutical aspects of the TTS are the drug release profile, distribution of the drug within the patch, drug solubility in the matrix, drug and/or patch stability, adhesiveness of the patch to the skin, smooth and complete removeability of the patch from the skin.

As these parameters are influencing each other it is difficult to develop a new patch just by amending one parameter.

Recently, it has been found that the systems as described above unfortunately show long-term stability problems. If rotigotine crystals are formed in the self adhesive matrix during long term storage, crystal growth can lead to reduced release rates of rotigotine with the risk eventually falling below the specified values.

Due to the occurrence of a new polymorphic form (form II) of the drug substance rotigotine crystal formation on the commercialized patches can take place. An attempt to modify the manufacturing process resulted only in very limited success. These patches still require cold storage until administration to the skin. This results inter alia in a more complicated application instructions for the rotigotine patch, like that the cooled drug product should be taken out of the refrigerator at least 1 hour before application and the adhesive on the patch has to be warmed up when the patch is applied

In general, it is well known in the art that stabilizing the amorphous state of a drug substance in a pharmaceutical dosage form including transdermal systems can be very difficult, if as in the case of rotigotine, the amorphous form is only metastable and easily converts into crystals. In such a case, the self adhesive matrix as a whole represents a metastable solid dispersion.

It is, therefore, an object of the present invention to provide a TTS comprising rotigotine which does not exhibit the above described drawbacks. In particular, the TTS should display an appropriate drug release profile combined with adequate stability and should be stable at room temperature.

### SUMMARY OF THE INVENTION

It is now surprisingly found that when used in a specific weight ratio to rotigotine, PVP is unexpectedly able to stabilize the non-crystalline form of rotigotine and prevent rotigotine from re-crystallization in a solid dispersion, in form of a self adhesive matrix of a transdermal therapeutic system, thereby imparting sufficient long term storage stability properties to the transdermal therapeutic system, preferably at room temperature, and without negatively influencing other relevant parameters of the TTS.
Room temperature or ambient as used in the present application is to be understood to apply to a range from 15°C to 25°C. In one embodiment the room temperature is in a range from 18°C to 22°C and in another embodiment it is about 20°C.

Based on this finding, the present invention provides a transdermal therapeutic system comprising a solid dispersion comprising a dispersing agent and a dispersed phase, said dispersed phase comprising rotigotine and polyvinylpyrrolidone, wherein the weight ratio of rotigotine to polyvinylpyrrolidone is 9:6, rotigotine is rotigotine free base, and the transdermal therapeutic system is prepared by a manufacturing process, which comprises preparing the solid dispersion, coating, drying and laminating to get the bulk product and converting the laminate into patch units via cutting and packaging.

In one embodiment, the solubility of rotigotine in the dispersing agent of the transdermal therapeutic system is below 1 wt-%.

In one embodiment, the dispersing agent of the transdermal therapeutic system comprises at least one silicone pressure sensitive adhesive.

In one embodiment, the dispersing agent of the transdermal therapeutic system comprises a mixture of a first silicone pressure sensitive adhesive and a second silicone pressure sensitive adhesive and the solid dispersion has a complex viscosity between 5 and 15 MP.

In one embodiment, rotigotine and polyvinylpyrrolidone are in a multitude of microreservoirs in the transdermal therapeutic system.

In one embodiment, the transdermal therapeutic system comprises 0.1 to about 3.15 mg/cm² of rotigotine in the free base form.

In one embodiment, rotigotine of polymorphic form II is employed as starting material in the manufacturing process of the transdermal therapeutic system.

### BRIEF DESCRIPTION OF DRAWINGS

Figure 1 shows the influence of varying rotigotine to polyvinylpyrrolidone weight ratios on the release of rotigotine from a TTS. API = active pharmaceutical ingredient (rotigotine); PVP = polyvinylpyrrolidone. "Specification" is related to the product specification of the existing Neupro^{®} Rotigotine patch.
Figure 2 shows the physical stability of rotigotine patches containing different amounts of PVP (rotigotine:PVP weight ratio of 9:2, 9:3 and 9:4) after 9 month at room temperature.
Figure 3 shows the physical stability of rotigotine patches containing different amounts of PVP (rotigotine:PVP weight ratio of 9:3 and 9:4) after 15 month at room temperature.
Figure 4 shows the physical stability of rotigotine patches containing different amounts of PVP (rotigotine:PVP weight ratio of 9:3 and 9:4) after 19 month at room temperature.

### DETAILED DESCRIPTION

Polyvinylpyrrolidone (PVP) is a polymer made from the monomer N-vinylpyrrolidone. It increases the cohesion of silicone adhesives. The molecular weight of the polyvinylpyrrolidone can be in the range from 2,000 to 2,500,000 Dalton (g/mol) (given as weight average), in one embodiment in the range from 700 000 to 1,500,000, in another embodiment in the range from 1,000,000 to 1,500,000 dalton. Various grades of PVP are commercially available from e.g. BASF Aktiengesellchaft of Ludwigshafen, Germany, e.g. under the name of Kollidon. For example, the following grades of Kollidons are water soluble forms of PVP: K-12 PF (molecular weight = 2,000 - 3,000); K-17 PF (molecular weight= 7,000 - 11,000); K-25 (molecular weight = 28,000 - 34,000); K-30 (molecular weight = 44,000 - 54,000); and K-90F (molecular weight = 1,000,000 - 1,500,000). In a preferred embodiment, the molecular weight of the polyvinylpyrrolidone is in the range from 28,000 to 1,500,000 Dalton (g/mol). Particularly preferred are the Kollidon grades K-25, K-30 and K-90F.

It is known from previous experiments that PVP reduces drug release from transdermal delivery systems with rotigotine across lipophilic membranes. Surprisingly it has been found, that a rotigotine/PVP weight ratio of 9:6, has only a very small effect on drug skin permeation but a highly significant impact on physical stability of the patch.

The present invention is based on the finding that when rotigotine and polyvinylpyrrolidone are employed in a weight ratio of 9:6, polyvinylpyrrolidone is able to stabilize solid dispersions of the non-crystalline form of rotigotine by preventing rotigotine from crystallization.

Thus, disclosed herein is the use of polyvinylpyrrolidone for the stabilization of a solid dispersion of non-crystalline form of rotigotine in a dispersing agent.

According to the present invention, polyvinylpyrrolidone was found to stabilize non-crystalline rotigotine in the solid dispersion state when the two components are used in a weight ratio of rotigotine to polyvinylpyrrolidone of 9:6.

Accordingly, a solid dispersion is disclosed herein, comprising rotigotine and polyvinylpyrrolidone, wherein the weight ratio of rotigotine to polyvinylpyrrolidone is 9:6.

It will be appreciated by the skilled person that any multiple of the above weight ratios will also be encompassed by the present invention. That is, the weight ratio of rotigotine to polyvinylpyrrolidone of 9:6 for example also encompasses the weight ratio of 18:12.

The term "stabilization" as used herein means that the non-crystalline form of rotigotine in a solid dispersion is maintained due to preventing rotigotine from crystallization over a certain period of time under defined conditions. In particular, a stabilization of at least 2 years under storage at room temperature or temperatures not exceeding 25°C is intended. This means that degree of rotigotine crystallization in the solid dispersion should not exceed 10 %, more preferably should not exceed 5 % and most preferably should not exceed 2 % (all percentages used herein are by weight, unless provided otherwise), based on the initial amount of rotigotine in the solid dispersion after 24 months storage in a sealed container at room temperature.

In one embodiment the non-crystalline form of rotigotine is amorphous rotigotine.

The term "solid dispersion" as used herein refers to a metastable system consisting of a dispersing agent and a dispersed phase, which is immiscible with the dispersing agent.

The dispersing agent of the solid dispersion may be any solid or semi-solid semi-permeable silicone-based polymer or copolymer or in another embodiment the dispersing agent is an acrylate. The dispersing agent should provide sufficient activity and stability for the solid dispersion as well as sufficient release of rotigotine.

Usually this polymer will be a pressure sensitive adhesive (PSA) or a mixture of such adhesives.

In one embodiment, the dispersing agent comprises at least one adhesive having a complex viscosity between 40 and 250 MP (mega-poise, i.e. 1 million poise; 1 P (poise) corresponds to 1 g/(cm*s) or 0.1 kg/(m*s), i.e. 0.1 Pa*s), in another embodiment between 50 and 200 MP and in still another embodiment between 60 and 150 MP and in a further embodiment the complex viscosity is between 70 and 120 MP.

It was surprisingly found that a sufficient physical long-term stability of the solid drug dispersion can be achieved by when the upper complex viscosity is below 250 MP and most preferably below 200 MP.

In a further embodiment, the dispersing agent comprises at least one second adhesive having a complex viscosity between 1 and 10 MP, in another embodiment between 1.5 and 5 MP and in still another embodiment between 2 and 3 MP.

In one embodiment the complex viscosity of the mixture of adhesives is between 5 and 25 MP. In another embodiment the complex viscosity of the mixture of adhesives is between 6 and 20 MP. In another embodiment the complex viscosity of the mixture of adhesives is between 7 and 15 MP and in still another embodiment between 8 and 12 MP.

In one further embodiment, the complex viscosity of the solid dispersion comprising a mixture of adhesives as dispersing agent and rotigotine and polyvinylpyrrolidone in the dispersed phase is between 5 and 15 MP, in another embodiment between 6 and 15 MP, in another embodiment between 8 and 15 MP and in still another embodiment between 10 and 12 MP.

Complex viscosity was determined by using a Stress Tech Rheometer (REOLOGICA Instruments AB or equivalent) being equipped with plate/plate measurement system with d = 25 mm and a temperature regulator capable to adjust the temperature between 15 and 110 °C. All rheology tests were done in the linear viscoelastic region of the test material at an oscillation frequency of 0.1 rad/s and 30 °C. Depending on the complex viscosity of the respective samples, the following parameters were used:
a) adhesives having a complex viscosity between 40 and 250 MP: thickness of the sample films: about 2 mm; strain: 5 %; gap: 1.5 mm; and
b) adhesives having a complex viscosity between 1 and 10 MP: thickness of the sample films: about 3 mm; strain: 1 %, gap: 2.5 mm.

Further disclosed herein is a solid dispersion comprising a mixture of adhesives as dispersing agent and rotigotine and polyvinylpyrrolidone in the dispersed phase, wherein the solid dispersion has a peel adhesion at a thickness of 50g/m² between 3 and 16 N/50 mm, in another embodiment between 4.5 and 14 N/50 mm and in still another embodiment between 6 and 12 N/50 mm, and/or has a peel adhesion at a thickness of 150g/m² between 14 and 26 N/50 mm, in another embodiment between 16 and 24 N/50 mm, in still another embodiment between 17 and 23 N/50 mm and in a further embodiment around 20.1 N/50 mm.

Peel adhesion was determined by using a tensile test machine (ZWICK/ROELL Z2.5 or equivalent). Samples were mounted on a suitable test plate made of aluminium and left for 10 min before peeling off. The sample films had a thickness of 50g/m² or of 150g/m². All measurements were done with a peel speed of 300 mm/min and a peel angle of 90° at room temperature.

Still disclosed herein is a solid dispersion comprising a mixture of adhesives as dispersing agent and rotigotine and polyvinylpyrrolidone in the dispersed phase, wherein the solid dispersion has a static shear adhesion between 20 and 150 min, in another embodiment between 25 and 100 min, in still another embodiment between 30 and 80 min and in a further embodiment the static shear adhesion is between 40 and 60 min.

Static shear adhesion was determined using a method based on DIN standard method EN 1943:2003-01 "Adhesive tapes, measurement of the shear force under static load". In brief, for the determination of the shear force, a defined area (12 mm x 12 mm) of a sample film was stuck on a test plate made of V2A steel and is rolled twice with constant pressure (roll: m = 5 kg, width = 2.5 cm) in longitudinal direction for ca. 1 sec. Afterwards, the test plate was vertically arranged and a standard weight (1000 ± 5 g) was attached at the free end of the sample. The time until failure of the adhesive sealing of the sample, i.e. the time until the weight drops down, was measured. All measurements were done at 23 ± 2 °C and 50 ± 5 % RH. Before, the samples were allowed to equilibrate under these standard conditions for at least 24 h.

The adhesives used in the present invention should preferably be pharmaceutically acceptable in a sense that they are biocompatible, non-sensitising and nonirritating to the skin of the recipient. Particularly advantageous adhesives for use in the present invention should further meet the following requirements:
1. retained adhesive and co-adhesive properties in the presence of moisture or perspiration, under normal temperature variations; and
2. good compatibility with rotigotine, as well as with the further excipients.

Although different types of pressure sensitive adhesives may be used in the present invention, it is preferred to use lipophilic adhesives having both low drug and low water absorption capacity. Preferably, the adhesives have solubility parameters which are lower than those of rotigotine. Such preferred pressure sensitive adhesives are amine-compatible silicone type pressure sensitive adhesives.

In a preferred embodiment the dispersing agent comprises at least one silicone pressure sensitive adhesive and preferably a mixture of at least one high tack and at least one medium tack silicone pressure sensitive adhesive.

Especially preferred pressure sensitive adhesives are of the type forming a soluble polycondensed polydimethylsiloxane (PDMS)/resin network, wherein the hydroxy groups are capped with e.g. trimethylsilyl (TMS) groups. Preferred adhesives of this kind are the BIO-PSA silicone pressure sensitive adhesives manufactured by Dow Corning, particularly the Q7-4201 and Q7-4301 qualities.

However, other silicone adhesives may also be used.

Tack has been defined as the property that enables an adhesive to form a bond with the surface of another material upon brief contact under light pressure (see e.g."Pressure Sensitive Tack of Adhesives Using an Inverted Probe Machine", ASTM D2979-71 (1982); H. F. Hammond in D. Satas "Handbook of Pressure Sensitive Adhesive Technology" (1989), 2nd ed., Chapter 4, Van Nostrand Reinhold, New York, page 38).

Medium tack of a silicone pressure sensitive adhesive indicates that the immediate bond to the surface of another material is weaker compared to a high tack silicone adhesive.

The mean resin/polymer ratio is approx. 60/40 for medium tack adhesives, whereas it is approx. 55/45 for high tack adhesives. It is known to the skilled person that both tape and rheological properties are significantly influenced by the resin/polymer ratio (K. L. Ulman and R. P. Sweet "The Correlation of Tape Properties and Rheology" (1998), Information Brochure, Dow Corning Corp., USA).

Blends comprising a high tack and a medium tack silicone type pressure sensitive adhesive comprising polysiloxane with a resin are advantageous in that they provide for the optimum balance between good adhesion and little cold flux. Excessive cold flux may be disadvantageous since it results in too soft solid dispersions.

Preferably, the weight ratio of a high tack to a medium tack silicone type pressure sensitive adhesive in these blends is 1:1. However, this does not exclude employing any other weight ratio.

A mixture of the aforementioned Q7-4201 (medium tack) and Q7-4301 (high tack) qualities proved to be especially useful for the preparation of a solid dispersion according to the present invention.

The solid or semi-solid semi-permeable polymer forming the dispersing agent has to satisfy the following requirements:
1. Sufficient solubility and permeability for the free base form of rotigotine.
2. Impermeability for the protonated form of rotigotine.

In one embodiment the solubility of rotigotine (without stabilizer) in the dispersing agent is about 5 wt-% or below and in another embodiment about 3 wt-% or below. In still another embodiment the solubility of rotigotine (without stabilizer) in the dispersing agent is about 2 wt-% or below and in another embodiment it is about 0.1 wt-% or below.

The dispersed phase of the solid dispersion comprises rotigotine in non-crystalline form and a stabilizer, for example polyvinylpyrrolidone, and optionally further pharmaceutically acceptable excipients, such as permeation enhancers and antioxidants. In one embodiment the stabilizer is selected from polyvinylpyrrolidone and in a preferred embodiment from water soluble polyvinylpyrrolidone. Copolymers of polyvinylpyrrolidone and vinyl acetate, polyethyleneglycol, polypropyleneglycol, glycerol and fatty acid esters of glycerol or copolymers of ethylene and vinylacetate might also be considered for such use.

Suitable permeation enhancers may be selected from the group of fatty alcohols, fatty acids, fatty acid esters, fatty acid amides, glycerol or its fatty acid esters, N-methylpyrrolidone, terpenes such as limonene, [alpha]-pinene, [alpha]-terpineol, carvone, carveol, limonene oxide, pinene oxide, 1,8-eucalyptol and most preferably ascorbyl palmitate.

Suitable antioxidants are sodium metabisulfite, ascorbylpalmitate and DL-alpha tocopherol.

Unless expressly indicated otherwise, any references to rotigotine in the context of this invention and the claims of this application mean rotigotine in the form of its free base. In some cases, however, traces of rotigotine hydrochloride may be contained in a rotigotine preparation but these traces typically do not exceed 5 wt-%, based on the amount of the free base. More preferably the content of hydrochloride impurities should be less than 2 wt-%, even more preferably less than 1 wt-% and most preferably the rotigotine used in the present invention contains less than 0.1 wt-% or no hydrochloride impurities at all.

A further step, which may be taken for reducing the amount of the salt form of rotigotine, is isolating the free base form of rotigotine in solid form prior to the preparation of the solid dispersion. If the free base of rotigotine is produced in situ during the manufacture of the solid dispersion by neutralizing an acid addition salt, a certain residue of the ionized drug form will remain (usually > 5 wt-% and up to approximately 10 wt-%). Therefore, such in situ preparation of the free base form will generally not be suitable for practising the present invention.

It will be understood by a person skilled in the art that rotigotine exists in various isomeric forms. It thus has also to be understood that any single isomer or a mixture of different isomers may be used in the present invention.

Hence, the S- or R-enantiomer or the racemate or any other enantiomeric mixture of rotigotine may be used.

In one embodiment of the invention the water content of the solid dispersion is less than 0.4 wt-% and in another embodiment it less than 0.2 wt-% related to the total patch matrix.

In one embodiment, high molecular weight polyvinylpyrrolidone (PVP) having a molecular weight of about 1 Mio Da is employed in the present invention. Rotigotine mixtures with these high molecular weight povidone grades result in a high degree of drug immobilization at room temperature. However, the glass temperature of mixtures of rotigotine/PVP with a weight ratio of 9:6 is low enough to enable sufficiently high drug release at skin temperature.

While not wishing to be bound by theory it is believed that free rotigotine is molecularly dispersed in the dispersing agent (outer phase) and that a non crystalline form of rotigotine is reversibly associated with PVP by forming an inner phase or microreservoir. In one embodiment the non-crystalline form of rotigotine is amorphous rotigotine. One advantage of a stable solid drug dispersion is that it can significantly reduce constraints often caused by low drug solubility in polymers suitable for transdermal delivery.

This does not exclude and will normally even imply that a certain fraction of rotigotine is dissolved in the dispersing agent of the solid dispersion at its saturation concentration.

The term "microreservoirs" as used herein is meant to be understood as particulate, spatially and functionally separate compartments consisting of a mixture of rotigotine and polyvinylpyrrolidone, which are dispersed in the dispersing agent of the solid dispersion. In one embodiment the solid dispersion contains 10³ to 10⁹ microreservoirs per cm² of its surface, in another embodiment this in the range of 10⁶ to 10⁹ microreservoirs per cm².

The maximum diameter of the microreservoirs is less than the thickness of the solid dispersion, preferably up to 85 % of the thickness of the solid dispersion, particularly preferably 5 to 74 % of the thickness of the solid dispersion. For an exemplary thickness of the solid dispersion of 50 µm this corresponds to a maximum diameter of the microreservoirs in the range of preferably up to approx 40 to 45 µm.

The term "maximum diameter" as used herein is meant to be understood as the diameter of the microreservoirs in one dimension (x-, y-, or z-dimension), which is the largest. It is clear to the skilled person that in case of spherical diameters the maximum diameter corresponds to the microreservoir's diameter. However, in the case of microreservoirs, which are not shaped in the form of spheres, i.e. of different geometric forms-, the x-, y- and z-dimensions may vary greatly.

In a particularly preferred embodiment of the invention, the mean diameter of the rotigotine containing microreservoirs distributed in the solid dispersion is in the range of 1 to 40 %, even more preferably 1 to 20 %, of the thickness of the solid dispersion. For an exemplary thickness of the solid dispersion of 50 µm this corresponds to a mean diameter of the microreservoirs in the range of preferably 0.5 to 20 µm.

The term "mean diameter" as used herein is defined as the mean value of the x, y, z-average diameters of all microreservoirs. The target particle size can be adjusted by the solids content and the viscosity of the solid dispersion.

The maximum and mean diameters of the microreservoirs as well as the number of microreservoirs per surface area of the solid dispersion can be determined as follows: The surface of the solid dispersion is examined with a light microscope (Leica microscope type DM/RBE equipped with a camera type Basler A 113C). The measurement is performed by incidental polarized light analysis using a microscope at 200x magnification. A picture analysis is performed using the software Nikon LuciaDi, Version 4.21, resulting in mean and maximum diameters for each sample.

The solid dispersion disclosed herein represents a self adhesive matrix of a transdermal therapeutic system.

Thus, disclosed herein is the use of a solid dispersion as specified above for the preparation of the transdermal therapeutic system of the present invention, which comprises the solid dispersion as self adhesive matrix.

The present invention relates to a transdermal therapeutic system comprising a solid dispersion as specified above as self adhesive matrix.

The term "transdermal therapeutic system" (TTS) as used herein refers to a matrix-type patch having a continuous self adhesive matrix in at least its center portion, which matrix is formed by the above specified solid dispersion. Such a patch consists of a backing layer, the self adhesive matrix and a protective foil or sheet, which is removed before use.

The backing layer is inert to the components of the self adhesive matrix. It is a film being impermeable to rotigotine. Such a film may consist of polyester, polyamide, polyethylene, polypropylene, polyurethane, polyvinyl chloride or a combination of the aforementioned materials. These films may or may not be coated with an aluminum film or with aluminum vapour. The thickness of the backing layer may be between 10 and 100 µm, preferably between 15 and 40 µm.

The self adhesive matrix layer formed by the above specified solid dispersion may have a coating weight between 50 and 150 g/m², preferably between 50 and 75 g/m² and most preferably is 50 g/m² ± 5 %.

Sporadically few tiny spots or bubbles may be observed in the adhesive matrix of some patches. They are small areas where the backing film can be seen through a small hole in the adhesive matrix, and thus are referred to as "matrix-free spots". In general these spots do not occur and their occurrence does, moreover, not have any impact on the pharmaceutical quality of these patches.

In a particular preferred embodiment, the self-adhesive matrix is free of particles, which can absorb salts of rotigotine on the TTS/skin interface. Examples of particles, which can absorb salts of rotigotine on the TTS/skin interface, include silica. Such particles, which can adsorb salts of rotigotine, may represent diffusion barriers for the free base form of the drug and may result in the formation of channels inducing some permeability of the self-adhesive matrix for the protonated form of rotigotine, which is disadvantageous.

Preferably, the TTS contains less than 1 wt-% of inorganic silicates, most preferably it is completely free from inorganic silicates.

The protective foil or sheet will be removed immediately prior to use, i.e. immediately before the TTS will be brought into contact with the patient's skin. The protective foil or sheet may consist of polyester, polyethylene or polypropylene, which may or may not be coated with aluminum film or aluminum vapour or fluoropolymers. Typically, the thickness of such a protective foil or sheet ranges between 50 and 150 µm.

So as to facilitate removal of the protective foil or sheet when wishing to apply the TTS, the protective foil or sheet may comprise separate protective foils or sheets having overlapping edges, similar to the kind used with the majority of conventional plasters.

In one embodiment, the TTS has a basal surface area of 0.5 to 50 cm², and another one of about 1 to 50 cm² and in still another one of about 5 to 50 cm². In another embodiment the TTS has a basal surface area of 10 to 40 cm², and in another one of about 10 to 30 cm² and in another one of about 20 to 30 cm². It goes without saying that a device having a surface area of, say, 20 cm² is pharmacologically equivalent to and may be exchanged by two 10 cm² devices or four 5 cm² devices having the same drug content per cm². Thus, the surface areas as indicated herein should be understood to refer to the total surface of all devices simultaneously administered to a patient.

Providing and applying one or several TTS has the pharmacological advantage over oral therapy that the attending physician can titrate the optimum dose for the individual patient relatively quickly and accurately, e.g. by simply increasing the number or size of devices given to the patient. Thus, the optimum individual dosage can often be determined after a time period of only about 3 weeks with low side effects.

A preferred content of rotigotine in the TTS is in the range of 0.1 to 3.15 mg/cm². Still more preferred are 0.4 to 1.5 mg/cm² and 0.2 to 1.0 mg/cm². If a 7 day patch is desired, higher drug contents will generally be required. A rotigotine content in the range of about 0.4 to 0.5 mg/cm² has been found to be particularly advantageous in that it provides the optimum usage of the drug contained in a once-a-day TTS, i.e. there is only very little residual drug content in the TTS after administration. In a particular preferred embodiment, the rotigotine content is 0.45 mg/cm². The apparent dose administered in 24 hours by using such a TTS usually is 40 to 50 % and may be intraindividually as high as 80-90 % of the drug amount originally contained in the TTS. In general, the content of rotigotine in the TTS can be adapted accordingly in order to provide a suitable flux for a multiple day TTS.

The TTS of the present invention is prepared by a manufacturing process, which comprises preparing a rotigotine comprising solid dispersion, i.e. the self adhesive matrix, coating, drying and laminating to get the bulk product, converting the laminate into patch units via cutting, and packaging.

After preparation, the rotigotine-comprising solid dispersion contains rotigotine to a little extent in dissolved form and mainly in amorphous form. However, for preparing the solid dispersion either of the two crystalline forms of rotigotine, i.e. polymorphic form I or polymorphic form II, may be employed as a starting material. The manufacturing process is described in more detail below.

The water content in the TTS obtained after preparation is in general low enough so that no evaporation of water during preparation of the TTS is necessary. Typically, the water content in a freshly prepared patch is below 2 wt-%, in another embodiment more preferably 1 wt-% or lower and in another embodiment 0.6 wt-% or lower, depending on the drug/PVP weight ratio.

In a particularly preferred embodiment, the TTS is a silicone-based transdermal therapeutic system comprising a mixture of at least one high tack and at least one medium tack amine-compatible silicone pressure sensitive adhesive as the main adhesive components, about 0.1 to 3.15 mg/cm² of rotigotine in the free base form as active ingredient, and polyvinylpyrrolidone, wherein the weight ratio of rotigotine to polyvinylpyrrolidone is 9:6.

In a preferred embodiment rotigotine and polyvinylpyrrolidone are contained in said silicone-based transdermal therapeutic system in a multitude of microreservoirs.

The transdermal therapeutic systems of the present invention can be used as a medicament.

In a preferred embodiment, the medicament is used in the treatment of diseases susceptible to the action of dopamine receptor agonists, such as rotigotine.

Diseases, which may be treated include Parkinson's disease, Parkinson's plus syndrome, depression, the restless-legs syndrome and pain.

Also, the medicament may be used in the treatment or prevention of dopaminergic neurone loss.

It should be understood that the term "treatment" in the context of this application is meant to designate a treatment or an alleviation of the symptoms. The treatment may be of a therapeutic or prophylactic nature.

The invention and the best mode for carrying it out will be explained in more detail in the following non-limiting examples.

### EXAMPLES

Example 1: Influence of the rotigotine to PVP weight ratio on the crystallization of rotigotine in a TTS

### SAMPLE PREPARATION

Samples for testing the stabilizing properties of several rotigotine to polyvinylpyrrolidone weight ratios in terms of the present invention were manufactured basically following the process below.

5 g Sodium metabisulfite were dispensed in 45 g water while stirring until a clear 10% w/w/ aqueous solution is obtained.

### PVP SOLUTION

Polyvinyl pyrrolidone (Kollidon 90 F) (5 g) was added to 14.8 g anhydrous ethanol while stirring and leave the solution for swelling. After swelling the mixture is stirred until the polyvinyl pyrrolidone is entirely dissolved. After the complete dissolution of the polyvinyl pyrrolidone, an aqueous 10% w/w sodium metabisulfite solution (0.022 g), 0.05 g ascorbyl palmitate and 0.124 g all-rac-α-tocopherol are added. The mixture is stirred until a clear ethanolic PVP solution is obtained.

### PVP-ROTIGOTINE-SOLUTION

An ethanolic PVP solution as described above (15.998 g) are dissolved in anhydrous ethanol (42.33 g) and the mixture is heated to 30 - 40° C while stirring. Then (-)-5,6,7,8-tetrahydro-6-[propyl-[2-(2-thienyl)ethyl]-amino]1-naphthalenol (rotigotine, 18 g, polymorphic form I) was added to this solution. The mixture is stirred and heated to 50 - 60° C.

105.75 g of an amine resistant high tack silicone adhesive (BIO-PSA^{®} Q7-4301 mfd. by Dow Corning) (70 wt-% solution in heptane), 105.72 g of an amine resistant medium tack silicone adhesive (BIO-PSA^{®} Q7-4201 mfd. by Dow Corning) (70 wt-% solution in heptane), 63.54 g of the PVP-rotigotine-solution obtained above were mixed and all components were stirred until a homogenous dispersion was obtained.

The dispersion was coated onto a suitable fluoropolymer coated polyester release liner (e.g. SCOTCHPAK^{®} 9744) with a suitable doctor knife and the solvents were continuously removed in a drying oven at temperatures up to 80°C for about 30 min to obtain a drug-containing adhesive matrix of 50 g/m² coating weight. The dried matrix film was laminated with a polyester-type backing foil (e.g. Hostaphan MN 19). The individual patches were punched out of the complete laminate in the desired sizes (e.g. 10 cm², 20 cm², 30 cm²) and sealed into pouches under the flow of nitrogen.

Table 1 shows the composition in mg/20 cm² of the thus obtained rotigotine patch, which comprises rotigotine and polyvinylpyrrolidone in a weight ratio of 9:2 (comparative example)

**Table 1 Composition in mg/20 cm² of rotigotine patch 9:2 (comparative example)**

| **Component** | **Amount (mg)** |
|---|---|
| Rotigotine Base | 9.00 |
| Polyvinylpyrrolidone | 2.00 |
| Silicone BIO-PSA^{®} Q7-4301 | 44.47 |
| Silicone BIO-PSA^{®} Q7-4201 | 44.46 |
| Ascorbyl palmitate | 0.02 |
| DL-alpha Tocopherol | 0.05 |
| Sodium metabisulfite | 0.0006 |
| Matrix coating weight | 50 g/m² |

The above manufacturing process was adapted to the varying amounts of polyvinylpyrrolidone and the slightly increased coating weight of sample Nos. 1-8 (cf. Table 2 below; sample Nos. 1-5 and 7-9 are comparative samples; sample No. 6 is a sample according to the invention).

The rotigotine to polyvinylpyrrolidone weight ratio of 9:2 of comparative sample No. 1 corresponds to the rotigotine to polyvinylpyrrolidone weight ratio of Neupro^{®}.

During manufacturing of comparative sample No. 2 and comparative sample No. 3 disintegration tendencies of the suspension were observed during the coating process. That is, compositions based on rotigotine to polyvinylpyrrolidone weight ratios of 9:1 and 9:1.6 were not processable.

The 9:11 formulation of comparative sample No. 8 was only processable under heating during mixing the rotigotine/PVP solution with the adhesives and coating due to the high viscosity resulting from the high amount of PVP.

Based on the rotigotine to polyvinylpyrrolidone weight ratio of 9:4 of comparative sample 5, the above manufacturing process was further adapted to a reduced content of rotigotine and polyvinylpyrrolidone as well as an amended ratio of the used silicone adhesives and an increased matrix coating weight (comparative sample 9):

5.000 kg polyvinylpyrrolidone (Kollidon F 90) were dissolved in 33.81 kg anhydrous ethanol and the obtained solution was mixed with 0.0238 kg of an aqueous sodium bisulfite solution (10 % w/w), 0.0367 kg ascorbyl palmitate and 0.09291 kg DL-α-tocopherol.

18.033 kg of the thus obtained solution were heated to 30 - 40 °C while stirring and 5.199 kg crystalline rotigotine free base (polymorphic form II) were added to this solution in several portions. The mixture was stirred and further heated up to 50 - 60 °C for approximately 1 - 2 hours to completely dissolve the drug.

18.764 kg of the ethanolic drug / polyvinylpyrrolidone solution were added to a mixture of 60.83 kg BIO-PSA Q7 4301 (70 wt-% solution in heptane) and 30.41 kg BIO-PSA Q7 4201 (70 wt-% solution in heptane) and it was stirred for at least 1 hour until a homogenous dispersion was obtained.

The dispersion was coated onto a fluoropolymer coated release liner and the solvents were continuously removed in a drying oven at temperatures ranging from 40 °C up to 115 °C to obtain a dry drug-containing adhesive matrix of 75 g/m² coating weight. The dried matrix film was laminated with a polyester-type backing foil being siliconized on the inner side and aluminium vapor coated on the opposite side. The individual patches were punched out of the complete laminate and were sealed into pouches under nitrogen flow.

**Table 2 Rotigotine to polyvinylpyrrolidone weight ratios and coating weights of sample Nos. 1-9 (sample Nos: 1-5 and 7-9 are comparative samples; sample No. 6 is a sample according to the invention).**

| **Sample No.** | **1** | **2** | **3** | **4** | **5** | **6** | **7** | **8** | **9** |
|---|---|---|---|---|---|---|---|---|---|
| **Rotigotine:PVP [weight ratio]** | 9:2 | 9:1 | 9:1.6 | 9:3 | 9:4 | 9:6 | 9:8 | 9:11 | 9:4 |
| **Coating weight [g/m²]** | 60 | 62 | 62 | 61 | 59 | 63 | 61 | 63 | 75 |

### ANALYTICAL METHODS

Drug release was determined using Apparatus 5 (Paddle Over Disk) as described in the United States Pharmacopeia (USP 31-NF 26), Chapter 724 "Drug Release". United States Pharmacopeia Convention, Inc.: Rockville, Md, 2008 using the following conditions: dissolution medium: 900 ml phosphate buffer pH 4.5; temperature adjusted to 32±0.5°C; paddle rotation speed: 50 rpm; sampling times: 0.25, 0.5, 1, 2 and 3 h, respectively.

The released drug amounts were determined by a validated RP HPLC -method at a detection wave length of 272 nm.

Stability testing was performed with one patch of each sample. The tests were performed from 0 weeks up to 8 weeks for samples 1 to 9 and up to 24 months for further patch samples of a rotigotine:PVP weight ratio of 9:2, 9:3 and 9:4 after the production date under open storage conditions of 25 °C/60 % RH and the occurrence of crystals was recorded by taking microscopic pictures. These results were confirmed by DSC for selected samples.

### ANALYTICAL RESULTS

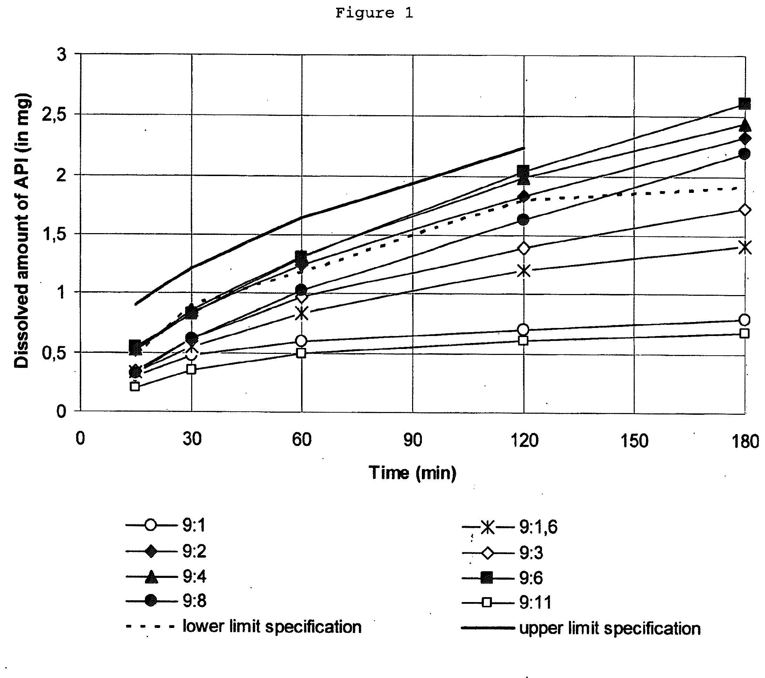

Results from drug release testing of samples 1-8 are shown in Figure 1.

Only sample Nos. 1 (comparative), 5 (comparative) and 6 (invention) meet the specifications set for drug release of the marketed Neupro^{®} patch having a comparable matrix thickness. *In vitro* drug release of comparative sample 9 (not shown) having a reduced content of rotigotine and polyvinylpyrrolidone and an increased matrix thickness also meets the Neupro^{®} specification after 3 hours, i.e. the endpoint of the test. For all remaining samples, the amount of rotigotine, which is released from the respective TTS during release testing is less than the specified lower limit.

Results from microscopic inspection of sample Nos. 1-9 are shown in Table 3.

For the rotigotine to polyvinylpyrrolidone weight ratios of 9:1 and 9:1.6 undissolved rotigotine crystals could microscopically be determined already in the freshly manufactured product. This finding could be confirmed by DSC measurements and reflects the problems occurring during manufacturing described above for these samples.

Starting with the first week crystals were observed for the rotigotine to polyvinylpyrrolidone weight ratios of 9:2 and 9:3.

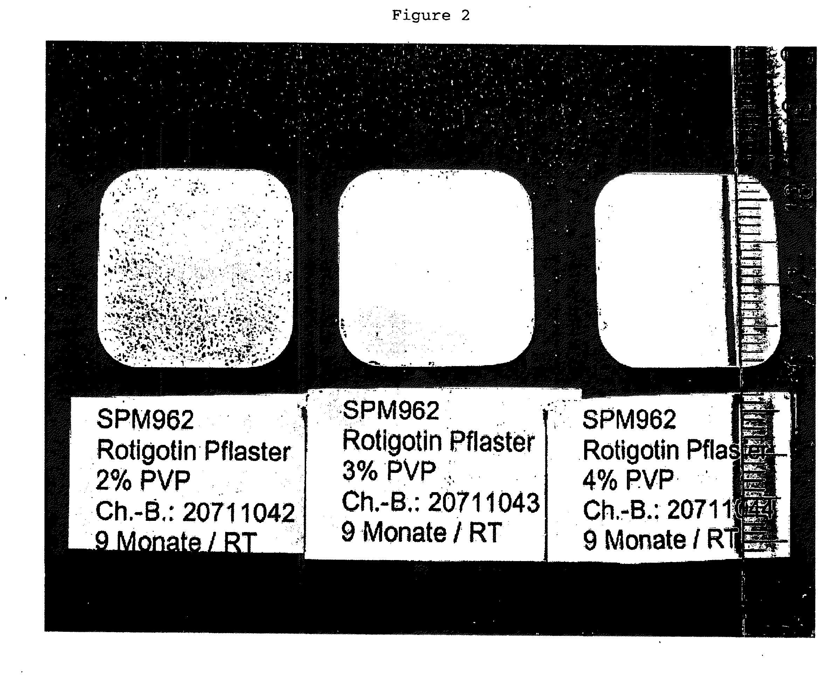

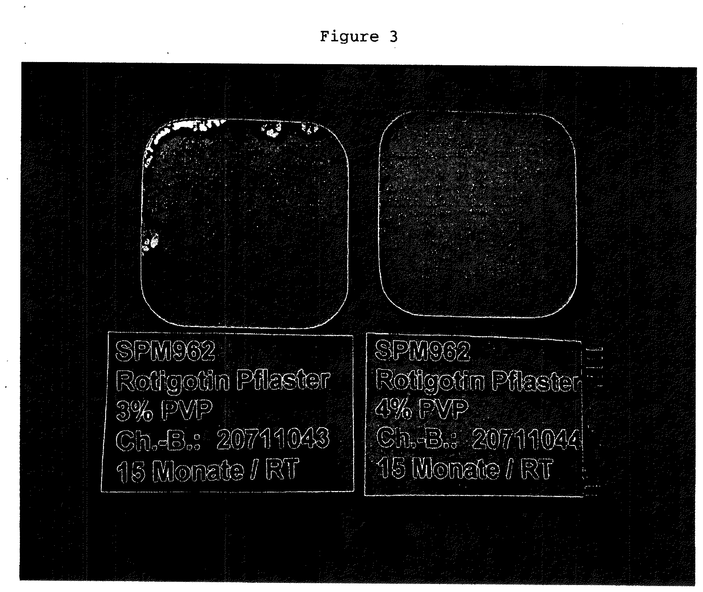

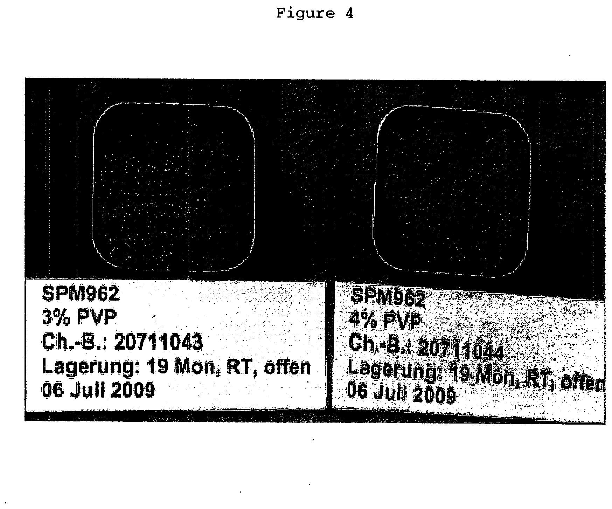

Figures 2 to 4 show that during long term stability testing of patches comprising different ratios of rotigotine to polyvinylpyrrolidone at room temperature an increasing amount of crystals was observed for the rotigotine to polyvinylpyrrolidone weight ratios of 9:2 and 9:3, whereas no crystal formation was observed for the rotigotine to polyvinylpyrrolidone weight ratio of 9:4 (comparative sample 5).

During long term testing of patches with a rotigotine to polyvinylpyrrolidone weight ratio of 9:4 (comparative sample 5) for up to 24 months at room temperature no crystals were observed.

Also comparative sample 9 with a rotigotine to polyvinylpyrrolidone weight ratio of 9:4 and having a reduced content of rotigotine and polyvinylpyrrolidone and an increased matrix thickness did not show any crystallization phenomena after long-term storage for up 18 months at 25°C.

An increasing PVP content delayed crystal appearance as it is shown for the rotigotine to polyvinylpyrrolidone weight ratios of 9:4 up to 9:11.

**Table 3 Results from storage stability testing of sample Nos. 1-9 at 25 °C/60 % RH (sample Nos. 1-5 and 7-9 are comparative samples; sample No. 6 is a sample according to the invention; + = crystals, - = no crystals)**

| **Sample No.** | **1** | **2** | **3** | **4** | **5** | **6** | **7** | **8** | **9** |
|---|---|---|---|---|---|---|---|---|---|
| **Rotigotine:PVP [weight ratio]** | 9:1 | 9:1.6 | 9:2 | 9:3 | 9:4 | 9:6 | 9:8 | 9:11 | 9:4 |
| **0 weeks** | + | + | - | - | - | - | - | - | - |
| **1 week** | + | + | + | + | - | - | - | - | - |
| **4 weeks** | + | + | + | + | - | - | - | - | - |
| **8 weeks** | + | + | + | + | - | - | - | - | - |

That is, taking also the above discussed data from release testing into account, optimum results could be achieved with rotigotine to polyvinylpyrrolidone weight ratios between 9:4 and 9:6. Higher ratios failed to sufficiently prevent rotigotine from crystallization and made the system prone to crystal growth or were even unprocessable. Lower ratios led to insufficient drug release by reducing the amount of rotigotine, which is released from the patch, below those values specified for the marketed Neupro^{®} patch.

## Claims

1. A transdermal therapeutic system comprising a solid dispersion comprising a dispersing agent and a dispersed phase, said dispersed phase comprising rotigotine and polyvinylpyrrolidone, wherein the weight ratio of rotigotine to polyvinylpyrrolidone is 9:6, rotigotine is rotigotine free base, and the transdermal therapeutic system is prepared by a manufacturing process, which comprises preparing the solid dispersion, coating, drying and laminating to get the bulk product and converting the laminate into patch units via cutting and packaging.

2. The transdermal therapeutic system of Claim 1, wherein the solubility of rotigotine in the dispersing agent is below 1 wt-%.

3. The transdermal therapeutic system of Claim 1 or 2, wherein the dispersing agent comprises at least one silicone pressure sensitive adhesive.

4. The transdermal therapeutic system of Claim 1, 2 or 3, wherein the dispersing agent comprises a mixture of a first silicone pressure sensitive adhesive and a second silicone pressure sensitive adhesive and wherein the solid dispersion has a complex viscosity between 5 and 15 MP as determined by a Stress Tech Rheometer.

5. The transdermal therapeutic system of Claim 1, 2, 3, or 4, wherein rotigotine and polyvinylpyrrolidone are in a multitude of microreservoirs.

6. The transdermal therapeutic system of Claim 1, 2, 3, 4 or 5, comprising 0.1 to about 3.15 mg/cm² of rotigotine in the free base form.

7. The transdermal therapeutic system of Claim 1, 2, 3, 4, 5 or 6, wherein in the manufacturing process rotigotine of polymorphic form II is employed as starting material.

## Patentansprüche

1. Transdermales therapeutisches System, umfassend eine feste Dispersion, die ein Dispersionsmittel und eine dispergierte Phase umfasst, wobei die dispergierte Phase Rotigotin und Polyvinylpyrrolidon umfasst, wobei das Gewichtsverhältnis von Rotigotin zu Polyvinylpyrrolidon 9:6 beträgt, Rotigotin die freie Base von Rotigotin ist und das transdermale therapeutische System durch ein Herstellungsverfahren hergestellt wird, das das Herstellen der festen Dispersion, Beschichten, Trocknen und Laminieren, um das Bulkprodukt zu erhalten, und das Umwandeln des Laminats in Pflastereinheiten durch Schneiden und Verpacken umfasst.

2. Transdermales therapeutisches System nach Anspruch 1, wobei die Löslichkeit von Rotigotin in dem Dispersionsmittel unter 1 Gew.-% liegt.

3. Transdermales therapeutisches System nach Anspruch 1 oder 2, wobei das Dispersionsmittel mindestens einen Silikonhaftklebstoff umfasst.

4. Transdermales therapeutisches System nach Anspruch 1, 2 oder 3, wobei das Dispersionsmittel ein Gemisch aus einem ersten Silikonhaftklebstoff und einem zweiten Silikonhaftklebstoff umfasst und wobei die feste Dispersion eine komplexe Viskosität zwischen 5 und 15 MP aufweist, bestimmt mit einem Stress Tech Rheometer.

5. Transdermales therapeutisches System nach Anspruch 1, 2, 3 oder 4, wobei Rotigotin und Polyvinylpyrrolidon in einer Vielzahl von Mikroreservoiren vorliegen.

6. Transdermales therapeutisches System nach Anspruch 1, 2, 3, 4 oder 5, umfassend 0,1 bis etwa 3,15 mg/cm² Rotigotin in Form der freien Base.

7. Transdermales therapeutisches System nach Anspruch 1, 2, 3, 4, 5 oder 6, wobei in dem Herstellungsverfahren Rotigotin der polymorphen Form II als Ausgangsmaterial eingesetzt wird.

## Revendications

1. Système thérapeutique transdermique comprenant une dispersion solide comprenant un agent dispersant et une phase dispersée, ladite phase dispersée comprenant de la rotigotine et de la polyvinylpyrrolidone, dans lequel le rapport pondéral entre la rotigotine et la polyvinylpyrrolidone est de 9:6, la rotigotine est une base libre de rotigotine, et le système thérapeutique transdermique est préparé par un procédé de fabrication qui comprend la préparation de la dispersion solide, l'enduction, le séchage et le laminage pour obtenir le produit en vrac, et la conversion du stratifié en patchs unitaires par découpage et conditionnement.

2. Système thérapeutique transdermique selon la revendication 1, dans lequel la solubilité de la rotigotine dans l'agent dispersant est inférieure à 1 % en poids.

3. Système thérapeutique transdermique selon la revendication 1 ou la revendication 2, dans lequel l'agent dispersant comprend au moins un adhésif silicone sensible à la pression.

4. Système thérapeutique transdermique selon la revendication 1, 2 ou 3, dans lequel l'agent dispersant comprend un mélange d'un premier adhésif silicone sensible à la pression et d'un deuxième adhésif silicone sensible à la pression, et dans lequel la dispersion solide présente une viscosité complexe comprise entre 5 et 15 MP telle que déterminée par un rhéomètre Stress Tech.

5. Système thérapeutique transdermique selon la revendication 1, 2, 3 ou 4, dans lequel la rotigotine et la polyvinylpyrrolidone sont dans une multitude de microréservoirs.

6. Système thérapeutique transdermique selon la revendication 1, 2, 3, 4 ou 5, comprenant de 0,1 à environ 3,15 mg/cm² de rotigotine sous forme de base libre.

7. Système thérapeutique transdermique selon la revendication 1, 2, 3, 4, 5 ou 6, dans lequel, dans le procédé de fabrication, de la rotigotine de forme polymorphe II est utilisée comme matière première.
